# EUROPEAN PATENT SPECIFICATION

(11) **EP 4 416 259 B1**
(45) Date of publication and mention of the grant of the patent: **03.06.2026**
(21) Application number: 21960769.4
(22) Date of filing: 12.10.2021
(51) Int. Cl.: C12M 1/16, C12P 7/10, B02C 19/00, D21C 1/00, D21C 1/02, D21C 1/04, D21C 7/02, D21B 1/06, D21C 5/02, D21H 11/14

(54) **CELLULOSE HYDROLYSIS APPARATUS FOR USE WITH HIGH SOLIDS LOADING**
CELLULOSEHYDROLYSEVORRICHTUNG ZUR VERWENDUNG MIT HOHER FESTSTOFFBELADUNG
APPAREIL D'HYDROLYSE DE LA CELLULOSE POUR UTILISATION AVEC UNE CHARGE ÉLEVÉE DE SOLIDES

(43) Date of publication of application: 21.08.2024
(73) Proprietor: EPISOME BIYOTEKNOLOJIK URUNLER SANAYI TICARET ANONIM SIRKETI, 41400 Gebze / Kocaeli (TR)
(72) Inventor: BALABAN, Murat, Üsküdar/Istanbul (TR); DEMIR, Ersin, Kadikoy/Istanbul (TR)
(74) Representative: Sögüt, Onur Ömer
(86) International application number: PCT/TR2021/051040
(87) International publication number: WO 2023/063895

(56) References cited:
- WO-A1-2006/056838
- WO-A1-2015/007773
- WO-A1-2016/094594
- CN-A- 106 635 788
- CN-A- 107 670 804
- CN-A- 107 983 504
- CHEN YUAN; FAN DONGBIN; HAN YANMING; LI GAIYUN; WANG SIQUN: "Length-controlled cellulose nanofibrils produced using enzyme pretreatment and grinding", CELLULOSE, SPRINGER NETHERLANDS, NETHERLANDS, vol. 24, no. 12, 21 September 2017 (2017-09-21), Netherlands , pages 5431 - 5442, XP036362087, ISSN: 0969-0239, DOI: 10.1007/s10570-017-1499-z
- ONOIKO TATYANA: "Features of process of preliminary grind of cellulose-containing materials", E3S WEB OF CONFERENCES, vol. 104, 1 January 2019 (2019-01-01), pages 01012, XP093063820, DOI: 10.1051/e3sconf/201910401012

## Description

### Technical Field

The present invention relates to a chemical engineering apparatus for use in enzymatic hydrolysis, second generation biofuel production and biorefining. In particular, the present invention relates to a cellulose hydrolysis apparatus capable to process high solids loading.

### Background

Cellulose is a polysaccharide formed from glucose monomers. Polysaccharide molecules in pluralities form elongated fibres that constitute respective, macromolecular yarns. Cellulose molecules firmly bonded to each other form "crystalline cellulose". On the other hand, if the cellulose molecules are loosely bonded to each other when compared to the crystalline cellulose, the respective structure is named as "amorphous cellulose".

Water retention ability of amorphous cellulose is considerably higher when compared to that of the crystalline cellulose, because water molecules can easily enter in-between loosely bonded cellulose molecules (fibres) in a macromolecular yarn of amorphous cellulose. Furthermore, also enzymes can be easily introduced in-between such fibres of an amorphous cellulose yarn. Accordingly, the amorphous cellulose has a relatively low resistance against cellulase enzymes, when compared to that of crystalline cellulose.

Cellulose hydrolysis is used in second-generation bioethanol and biogas production processes. The cellulose hydrolysis can be chemical or enzymatic. Chemical hydrolysis employs acids such as sulphuric acid, whereas enzymatic hydrolysis makes use of various enzymes that degrade cellulose into its monomers.

Enzymatic hydrolysis of cellulose can be exemplified as follows: materials that contain cellulose fibres (e.g., straw), are subjected to chemical pre-treatment, and then (enzymatically) hydrolysed in an aqueous suspension in the presence of enzymes, e.g., for 24 to 72 hours at temperatures between 50°C and 55°C. This hydrolysis step is usually performed at a low extent of dry matter percentage, at which the suspension shows fluid behaviour. The suspension is subjected to mixing using mixers, and simultaneously, cellulose yarn particles that buoy in the suspension are separated therefrom. The cellulose molecules are hydrolysed into glucose, resulting in a molasses.

Paper sludge as a cellulosic waste is a non-fluid-like, highly viscous, material that includes cellulose fibres and yarns along with fillers. Paper sludge has a high extent of water retention capability. Due to its cellulose content, the paper sludge has an important potential for being used in biofuel production processes based on fermentation. Due to being an industrial waste that has to be disposed of, as a raw material, input cost attributed to paper sludge is considered to be zero.

Paper production process results in loose bonds between cellulose fibres; therefore, the cellulose yarns in paper sludge are exposed, allowing introduction of aqueous fluids in-between the fibres. Hence, paper sludge has a much higher extent of water retention ability when compared to other, ligneous or straw-based materials. This fact results in very high viscosities in aqueous suspensions of paper sludge, which precludes mixing in industrial conditions.

Paper sludge even shows solid-like behaviour when the dry matter content reaches or exceeds 20 wt.%. Due to difficulties in mixing, enzymatic hydrolysis of such solid-like paper sludge is extremely difficult.

More specifically;
- Solids load ratio are low in hydrolysis that take place in a liquid medium. For instance, a 1000 kg of total medium weight can include 900 kg of an aqueous carrier (that can contain buffering components, surfactants etc.), 99 kg of cellulose, and 1 kg of enzymes. Such systems can be considered to be liquid.
- Systems that have a higher solids load ratio can include 50 wt.% to 80 wt.% of water with regard to the total weight of a respective medium. Such systems employ much lower water content when compared to the above-mentioned liquid systems; and allow the use of volumetrically smaller reactors to hydrolyse the same amount of dry cellulose when compared to a respective liquid system.
- In hydrolysis systems with low solids (or: dry) loadings, the water retention ability of delignified cellulosic material such as water sludge is much higher. Therefore, the viscosity is very high even with 1 wt.% of dry matter content with regard to the total weight of the respective medium. Such viscosity demands a high extent of energy consumption in mixing the medium. Hydrolysis with said low solids loading require high-volume mixers. Furthermore, the solid content has to be kept low in order to enable the use of industrial mixers. These measures dramatically increase the energy consumption per unit solid matter content in the medium.
- In the case of low solids loading, mixers stir the water but the consumed mixer power cannot be directly and efficiently exerted onto the particles that are suspended in the respective medium. Thus, the torque and shear force of the mixer cannot be efficiently exerted onto the particles.
- Hydrogen bridges between the water and cellulose molecules allow the capability of water retention. On the other hand, the macromolecular structure of cellulose yarns is constituted via the hydrogen bonds between cellulose molecules; and this fact results in a recalcitrance, that is, these hydrogen bonds between cellulose molecules can hinder the introduction of water and enzymes in-between cellulose fibres in a yarn.
- In particular, due to the above-mentioned firm bonding between cellulose fibres, the interaction of crystalline cellulose with aqueous medium of a respective suspension occurs only in a superficial level. This results in a bulk movement of cellulose in the suspension, because the aqueous medium (or water itself) cannot penetrate through hydrogen bridges in-between cellulose fibres in crystalline cellulose yarns. Hence, enzymes in the aqueous medium cannot efficiently be introduced in-between said cellulose fibres, and the respective enzymatic reaction occurs mainly in a superficial level. To overcome this issue, the level of crystallinity in the cellulose can be decreased by various methods such as steam explosion, ammonia fiber expansion (AFEX) and diluted acid treatment. Such measures introduce an increased processing cost.

Accordingly, it is required to improve in processing equipment for use in cellulose hydrolysis.

### Summary

The primary object of the present application is to overcome the above-mentioned shortcomings of the prior art. Another object of the present invention is to propose an apparatus and method that enable the reduction in the amount of enzymes necessary per cellulose unit for effectively hydrolysing the same. A further object of the present invention is to propose an apparatus and method that enable the processing of paper sludge with a relatively high solid load, thereby decreasing energy and apparatus investment costs. An even further object of the present invention is to propose an apparatus and method that enable an increased cellulose hydrolysis capacity, with decreased residence time in hydrolysis reaction.

The present invention achieves said objects with the features that constitute the appended independent claims.

The present improvement provides an apparatus and method that introduce an effective grinding of cellulose fibres and yarns. By such grinding, the crystallinity of cellulose can be decreased or even eliminated. Hence the water retention and enzymes receiving capability of cellulose is increased. Accordingly, the surface contact between enzymes and cellulose is increased, thereby effectively increasing the hydrolysis rate. This fact enables a decreased residence time, decreased hydrolysis reactor size without compromising capacity, effectively hydrolysing high solid load cellulose wastes without increasing costs related to investment and operating. Hence, the following are achieved by the apparatus and method proposed herein:
- the reduction in the amount of enzymes necessary per cellulose unit for effectively hydrolysing the same;
- the processing of paper sludge with a relatively high solid load, thereby decreasing energy and apparatus investment costs; and
- an increased cellulose hydrolysis capacity, and in which the residence time in hydrolysis reaction is decreased.

### Brief description of figures

Fig.1 is perspective view of an exemplary apparatus according to the present invention, showing a state where the one or more cylinders are at a first position with regard to the channel.
Fig.2 is frontal view of the exemplary apparatus shown in Fig.1, along the flow direction.
Fig.3 shows a partial, perspective view of the apparatus shown in Fig.1 and Fig.2, omitting the channel to emphasize an exemplary carrier in communication with an exemplary plurality of cylinders.
Fig.4 is perspective view of the exemplary apparatus shown in Fig.1 to Fig.3, showing a state where the one or more cylinders are at a first position with regard to the channel.
Fig.5a and Fig.5b show partial, perspective views of the apparatus shown in Fig.4, from different aspects.

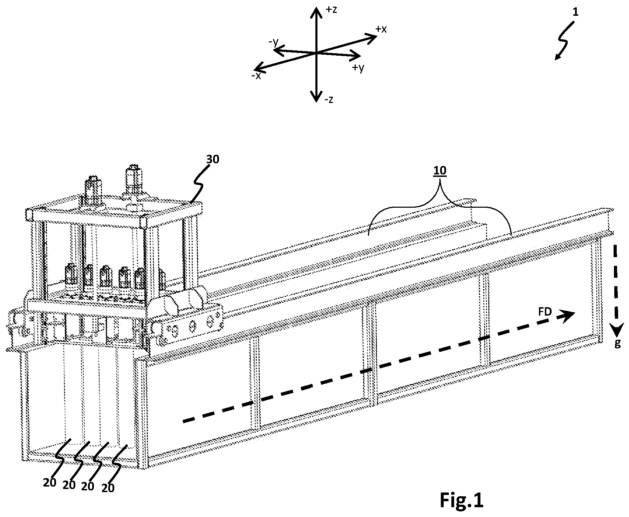

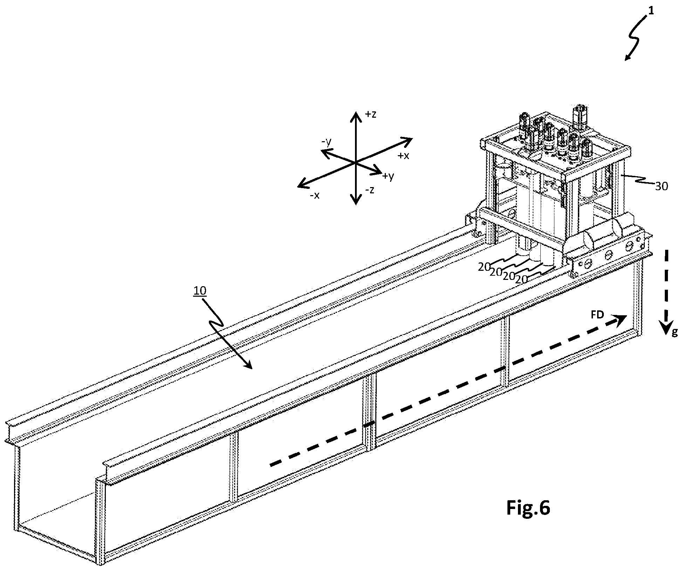

Fig.6 is perspective view of the exemplary apparatus shown in Fig.1 to Fig.5b, showing a state where the one or more cylinders are at a second position with regard to the channel, said second position being different from the first position. Here, the cylinders are retracted away from the channel, for avoiding contact between the cylinders and a respective waste stream. Such positioning can be achieved for instance prior to returning of the cylinders to an upstream end of the channel in accordance with the first translational movement, or at cleaning of the apparatus, or at an instance where the cylinders are just separated from the waste stream at a downstream end of the channel.
Fig.7 is perspective view of partial, perspective view of an apparatus according to the present invention, omitting the channel to emphasize an exemplary carrier in communication with an exemplary plurality of cylinders and plates arranged to provide complementary surfaces to side surfaces of respective cylinders.

### Detailed Description

With reference to the figures briefly described above, the present invention proposes an apparatus (1) for use in cellulose hydrolysis. The apparatus (1) comprises a channel (10) for guiding a stream of cellulose-containing waste in a flow direction (FD). The apparatus (1) further comprises one or more cylinder(s) (20) arranged to rotate around an axis (A), such that, when rotated, one or more side surfaces (21) of said cylinder(s) (20) exhibit a linear velocity vector orthogonal to said axis (A). The apparatus (1) further comprises one or more complementary surfaces that face said side surfaces (21), such that, when the cylinder(s) (20) are rotated, the side surfaces (21) and complementary surfaces cooperate in grinding the waste.

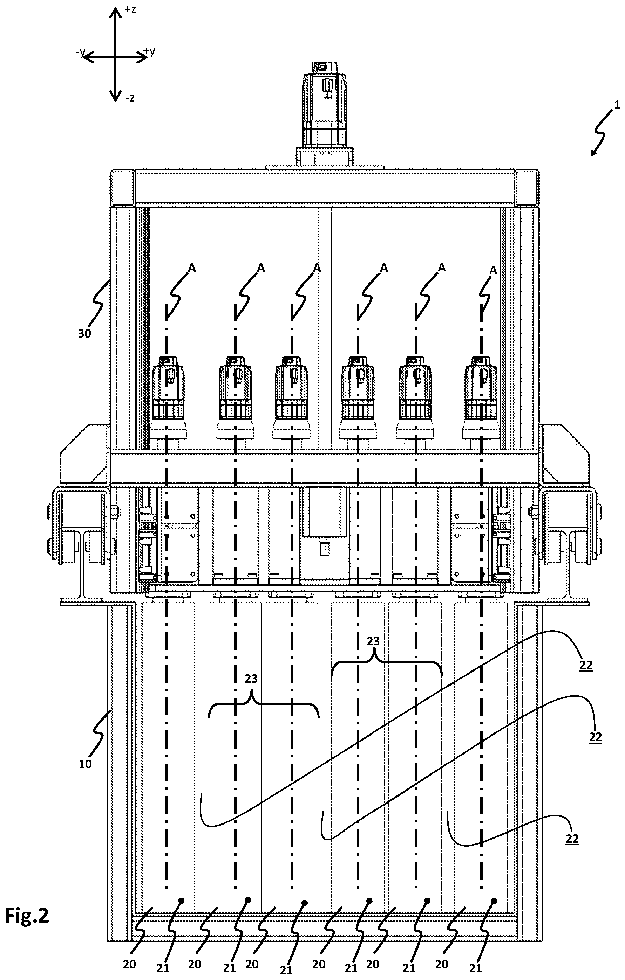

Fig.1 shows perspective view of an exemplary apparatus (1) according to the present invention, showing a state where the one or more cylinders (20) (here: a plurality thereof) are at a first position with regard to the channel (10). Here, the cylinders (20) are at a state where they are introduced in the channel (10), to contact and grind a respective waste stream in the channel (10) when the apparatus is in use. In other words, the cylinders (20) are positioned (here: lowered) for contacting a waste stream flowing through the channel (10) along the flow direction (FD). Fig.2 is frontal view of the exemplary apparatus shown in Fig.1, along the flow direction.

In accordance with the teaching above, the present invention further proposes a method for cellulose hydrolysis. The method can be performed for instance with the apparatus (1) described above. The proposed method includes the following actions:
- guiding a stream of cellulose-containing waste in a flow direction (FD) along a channel (10); and
- grinding the waste between one or more side surface(s) (21) of one or more cylinder(s) (20) that rotate around an axis (A) and one or more complementary surfaces that cooperate with respective side surface(s) (21), by arranging said side surface(s) (21) to exhibit a linear velocity vector orthogonal to the respective axis (A).

With these features, mechanical tension and pressure are simultaneously applied onto a respective cellulosic waste, to grind the same. Thus, any hydrogen bridges/bonds in-between cellulose yarns are easily loosened, and simultaneously, an enzyme-containing aqueous medium can be introduced into the cellulose. As a result, the proposed system (and the respective method) facilitates and expedites the cellulose hydrolysis reaction.

In a preferred embodiment of any of the above-discussed versions of the proposed apparatus (1), the axis (A) extends along the gravity vector (g) and orthogonal to the flow direction (FD) when the apparatus (1) is in use. Fig.1 to Fig.6 constitute examples to such embodiment.

In a preferred embodiment of any of the above-discussed versions of the proposed apparatus (1), the one or more cylinder(s) (20) are each arranged to rotate in a respective rotational direction(s). Thereby the ground waste is prompted in the flow direction (FD) guide the ground waste opposite to the flow direction (FD). Thus, the flow of the stream is promoted in an upstream direction (-x).

In a preferred embodiment of any of the above-discussed versions of the proposed apparatus (1), said one or more cylinder(s) (20) (e.g., each of them) are arranged to rotate such that respective side surfaces (21) thereof exhibit a linear velocity other than that of a corresponding complementary surface. In other words, the one or more cylinder(s) (20) are arranged to exhibit a relative linear velocity with respect to said side surfaces (21). Thus, the grinding is enhanced.

In an even more preferred embodiment of any of the above-discussed versions of the proposed apparatus (1), one or more pair(s) of cylinder(s) (20) adjacent to each other that are arranged such that their side surfaces (21) serve as complementary surfaces to each other, have respective diameters that are different from each other. With such embodiment, it is enabled that such pairs can be driven using a single motor in common, to rotate the cylinder(s) (20) at a single rotational speed (that is, at a single rpm value in common), yet achieving different linear velocities on respective side surfaces (21) of the cooperating cylinders (20) in the pair. Thus, a relative linear velocity (greater than zero) is achieved with regard to cooperating side surfaces (21) of said cooperating cylinders (20), with a simplified structure in the apparatus (1); enhancing the grinding effectivity, along with decreasing the costs.

An embodiment of any of the above-discussed versions of the proposed apparatus (1) can comprise one or more plate(s) (40) arranged to face said one or more cylinder(s) (20) for serving as complementary surface(s) to the side surfaces (21) of respective cylinder(s) (20). The one or more plate(s) (40) and the one or more cylinder(s) (20) can be provided on a carrier (30) to arranged that said plate(s) (40) and cylinder(s) (20) are translationally stationary relative to each other, whereas the cylinder(s) (20) are rotationally movable relative to the plate(s) (40). This version maximizes the grinding without necessitating mechanical driving forces to be applied to both of the respective bodies on which the side surfaces (21) and complementary surfaces are located (that is, onto the cylinder(s) (20) and plate(s) (40), respectively). Thus, a maximized extent of grinding can be achieved with a low extent of complexity in the apparatus (1), and with minimized extents of investment and operational costs. Such maximized extent of grinding also corresponds to a maximization and expediting of the cellulose hydrolysis.

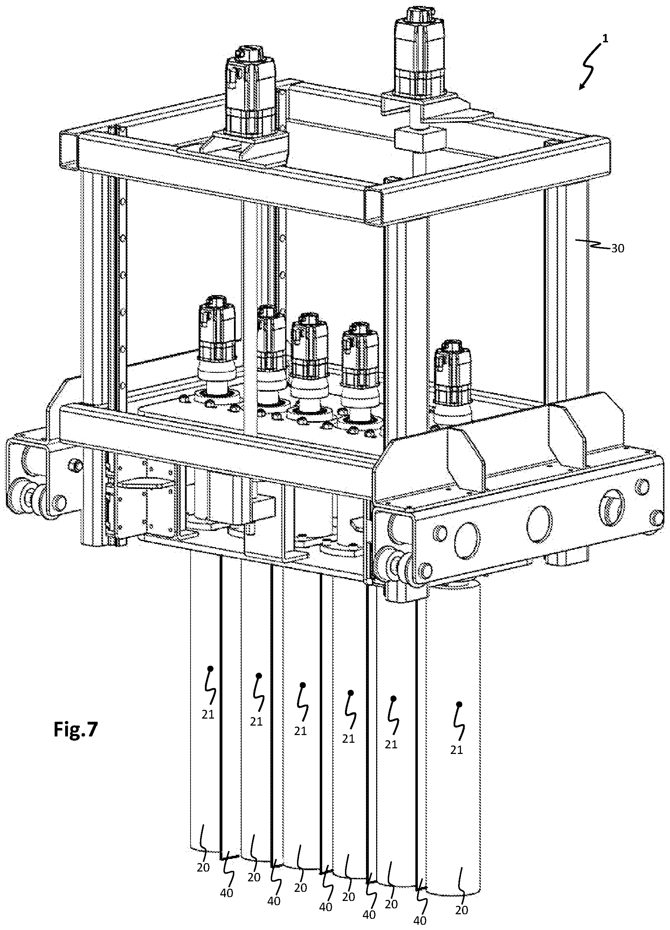

Fig.7 is perspective view of partial, perspective view of an exemplary apparatus (1) according to the present embodiment, omitting the channel (10) to emphasize an exemplary carrier (30) in communication with an exemplary plurality of cylinder(s) (20) and plate(s) (40) arranged to provide complementary surfaces to side surface(s) (21) of respective cylinders (20). Here, the cylinder(s) (20) and plate(s) (40) are alternatingly arranged such that a plate (40) is disposed in-between a respective pair of two consecutive cylinders (20) in a row. With such preferred embodiment, maximized extent of grinding (along with a maximization and expediting of the cellulose hydrolysis) can be achieved with a maximized number of stationary parts (here, plates (40)); thereby minimizing the complexity and production/operation costs of the apparatus (1).

Within the context of the present application, a row is to be considered as cylinders (and if applicable, plates) at an alignment along a lateral orientation (-y/+y) that is orthogonal to the flow direction (FD) and also to the gravity (g) when the apparatus (1) is in use.

Any of the embodiment of the apparatus (1) preferably comprises a plurality (e.g., two) of rows that are arranged along the flow direction (FD), thereby enabling a contact between an upstream-side row and the waste that is already contacted by a downstream-side row with regard to said upstream-side row.

Another embodiment of any of the above-discussed versions of the proposed apparatus (1) can comprise a plurality of cylinders (20) adjacent to each other, such that opposing side surfaces (21) of a pair of adjacent cylinders serve as complementary surfaces to each other. Preferably, the cylinders (20) that cooperate with each other in one or more pairs of said plurality of cylinders (20) can be arranged to rotate in opposite rotational directions, such that their side surfaces (21) opposing each other both have linear velocities opposite to the flow direction (FD). This measure locally promotes the waste to move in the upstream direction (-x), to guarantee that plurality of portions of the waste is ground only once at a grinding round; thereby enhancing the uniformity of hydrolysis throughout the channel (10). With the extent of grinding available with the present invention, this promotion of flow facilitates the removal of already and rapidly hydrolysed cellulose from the apparatus (1); thereby enhances the economic efficiency of the respective hydrolysis process. Fig.1 to Fig.6 constitute examples to such embodiment. Accordingly, in a preferred version of the method according to the present invention, the method includes coupling of opposing side surfaces (21) of a plurality of such cylinders (20) adjacent to each other, as complementary surfaces with regard to each other.

The plurality of cylinders (20) can be arranged in pairs, and a first distance between respective side surfaces (21) of two cylinders (20) that cooperate with each other within a pair can be arranged to have a first value (that is, comparatively narrow) to enable grinding of the waste; and a second distance between a pair and a further cylinder (20) in the same row adjacent to said pair can be arranged to be higher than the first distance (that enables said grinding), and a gap (22) is formed in-between the pair and the further cylinder (20) (that can be e.g., in another pair adjacent to said pair), so that waste can relatively easily pass through the gap (22) in an upstream direction opposite to the flow direction (FD) without being substantially ground. Fig.2 exemplifies the formation of gaps (22) in-between two pairs or in between a pair and a further cylinder (20) adjacent to said pair in the same row.

Considering that an embodiment of such apparatus (1) can comprise a plurality (e.g., two) of rows that are arranged along the flow direction (FD), thereby enabling a contact between an upstream-side row and the waste that is already contacted by a downstream-side row with regard to said upstream-side row:
- Preferably, the axes (A) of cylinders (20) in different rows are arranged to be offset with regard to the flow direction (FD). This feature enables the grinding of the waste that passes through the gap(s) in the downstream-side row, by a subsequent row.

In a preferred embodiment of any of the above-discussed versions of the proposed apparatus (1), the plurality of cylinders (20) adjacent to each other are arranged to exhibit different linear velocities with respect to the opposing side surfaces (21) of each other. In other words, the plurality of cylinders (20) adjacent to each other are arranged exhibit a relative linear velocity (that is, with a value different from zero) with respect to the opposing side surfaces (21) of each other. Accordingly, a preferred version of the method includes coupling of opposing side surfaces (21) of a plurality of such cylinders (20) adjacent to each other, as complementary surfaces with regard to each other. These corresponding measures further enhances grinding. Such enhanced extent of grinding also corresponds to an enhancement and expediting of the cellulose hydrolysis.

In a preferred embodiment of any of the above-discussed versions of the proposed apparatus (1), the side surfaces (21) of the one or more cylinders (20) (and if applicable, corresponding plate(s) (40)) are provided with indentations. With this measure, the side surfaces (21) serve in provision of roughness, thereby further enhancing the grinding. The roughness enhances the extent of grinding; thus, increases the efficiency and rate of the cellulose hydrolysis.

In a preferred embodiment of any of the above-discussed versions of the proposed apparatus (1), the apparatus (1) comprises a carrier (30) that is arranged to provide a first translational movement to the one or more cylinder(s) (20) relative to the channel (10) along the flow direction (FD). Referring to the appending drawings; within the context of the present invention, the flow direction (FD) corresponds to a downstream direction (+x) on a -x/+x orientation. So, alternating components of the reciprocations in first translational movement can be considered to be a movement in upstream direction (+x), and another movement in downstream direction (-x).

Accordingly, a preferred version of the method includes provision of a first translational movement to the one or more cylinder(s) (20) relative to the channel (10) along the flow direction (FD). With these corresponding measures attributed to the apparatus (1) and method according to the present invention, the one or more cylinder(s) (20) can be guided to exert the grinding along the channel (10). Thus, the uniformity in processing of the waste is enhanced. The first translational movement is to be considered as a first reciprocation that includes the following:
i. a translation of the one or more cylinder(s) (20) along the channel (10) in the flow direction (FW) (that is, in downstream direction (+x)) (e.g., whilst grinding of a respective waste), and then
ii. a transfer of said one or more cylinder(s) (20) back in an upstream direction (-x) to iterate said translation (e.g., a returning state for preparing to repeat the grinding state).

Fig.1 represents an exemplary apparatus (1) according to the present embodiment, and shows that the one or more cylinder(s) (20) are at an upstream end of the channel (10), and ready to be translated along the channel (10) in the flow direction (FW). On the other hand, Fig.6 represents such apparatus in which the one or more cylinder(s) (20) are ready to be transferred back in the upstream direction to iterate the grinding state.

In a preferred embodiment, the carrier (30) is arranged to provide that the first translational movement of the one or more cylinder(s) (20) is orthogonal to their respective axes (A). This feature enables the effective use of a smallest possible height in cylinder(s) (20) along respective axes (A).

In a preferred embodiment of any of the above-discussed versions of the proposed apparatus (1), the carrier (30) is arranged to provide a second translational movement to the one or more cylinder(s) (20) relative to the channel (10) orthogonal to the flow direction (FD). Accordingly, a preferred version of the method includes provision of a second translational movement to the one or more cylinder(s) (20) relative to the channel (10) orthogonal to the flow direction (FD). With these corresponding measures attributed to the apparatus (1) and method according to the present invention, the one or more cylinder(s) (20) can be kept away from the stream at transferring back in the upstream direction, thereby avoiding any unnecessary frictional losses and any obstruction of the waste stream flow in the flow direction (FD).

Referring to the appending drawings; within the context of the present invention, said second translational movement takes place in an -z/+z orientation that is orthogonal to the flow direction (FD) that is on the -x/+x orientation. So, when the apparatus (1) is in use, alternating components of the reciprocations in second translational movement can be considered to be a movement in an upwards direction (+z), and another movement in a downwards direction (-z).

Considering that a waste stream is supported by a bottom of the channel (10) against gravity (g); the second translational movement is to be considered as a second reciprocation that includes the following:
a) a temporary extraction of the cylinder(s) (20) from the channel, thus a temporary separation of the cylinder(s) (20) from the stream, by moving the cylinder(s) (20) in the upwards direction (+z) (that is, away from the bottom of the channel (10)); and then
b) a (re-)introduction of the cylinder(s) (20) into the channel, by moving the cylinder(s) (20) in the downwards direction (-z) (that is, towards the bottom of the channel (10)), thus into the stream.

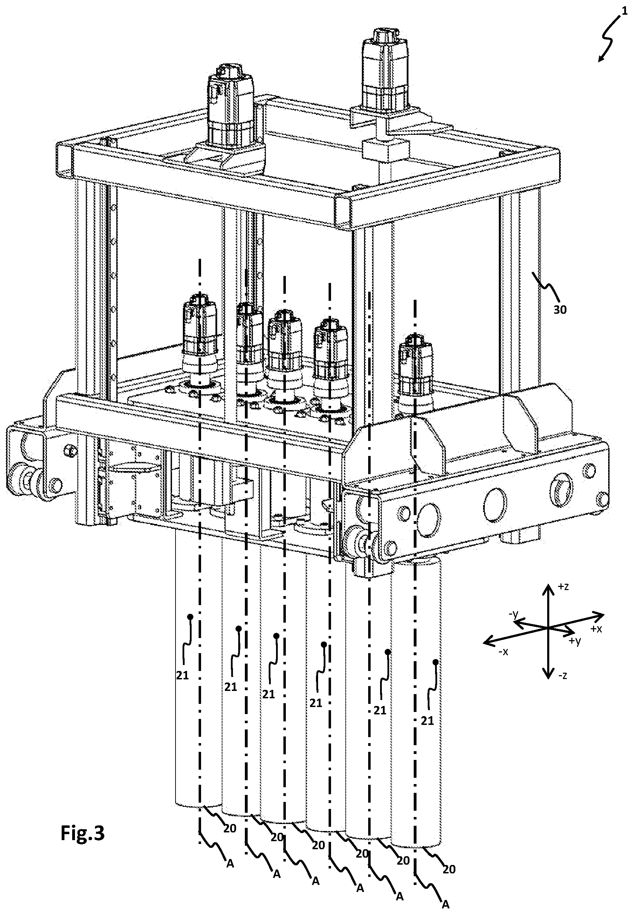

Within this context, Fig.1 and Fig.2 exemplify a positioning at a state (first position) where the one or more cylinder(s) (20) are introduced (or re-introduced) in the channel (10), for instance, by being extended from the carrier (30). Fig.3 shows a partial, perspective view of the apparatus shown in Fig.1 and Fig.2, omitting the channel (10) to emphasize an exemplary carrier (30) in communication with an exemplary plurality of cylinders (20). Here, the cylinders (20) are in a state where they are translated (here: introduced, extended or dipped) into the channel, in a direction orthogonal to the flow direction (FD), for instance, in accordance with the gravity (g). Thus, in such state, contact between the one or more cylinder(s) (20) and a respective waste stream is enabled.

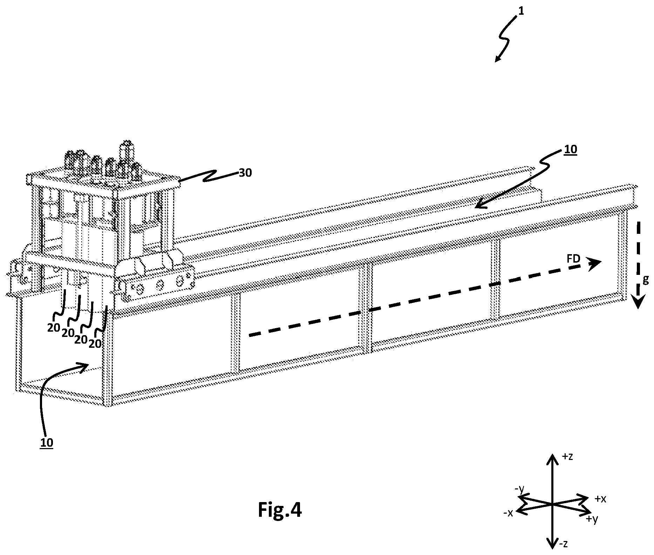

On the other hand, Fig.4 exemplifies a positioning at a state where the one or more cylinder(s) (20) are distanced from the channel (10) to separate the cylinder(s) (20) from a respective waste stream, for instance, by being retracted by the carrier (30). Here, the cylinder(s) (20) are positioned (here: elevated) to be separate/extracted from a waste stream flowing through the channel (10). Such extraction can be performed, for instance, by retracting the cylinder(s) (20) into the carrier (30). Such positioning can be achieved for instance prior to commencement of a hydrolysis procedure, or at cleaning of the apparatus (1), or at an instance where the cylinder(s) (20) are just returned from a downstream end of the channel (10) to be reintroduced into the waste stream at an upstream end of the channel (10).

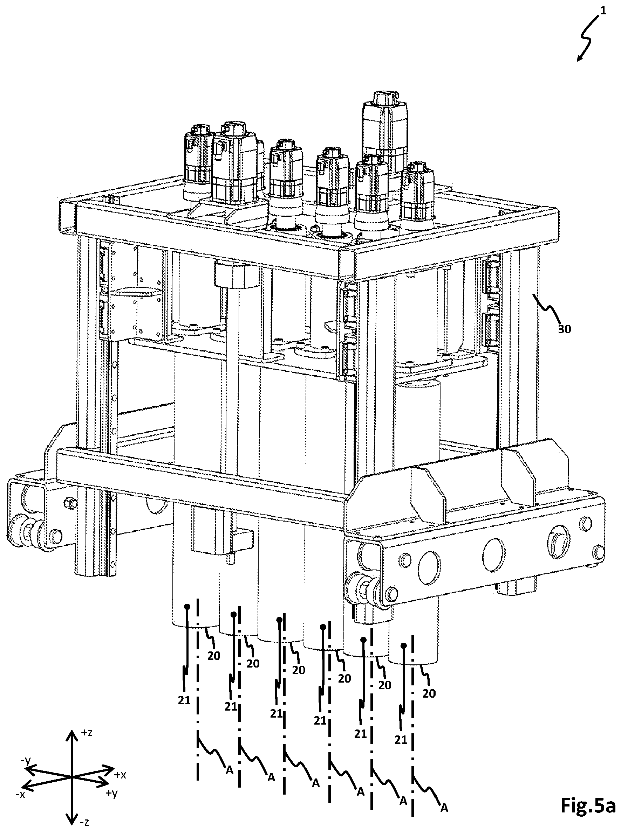

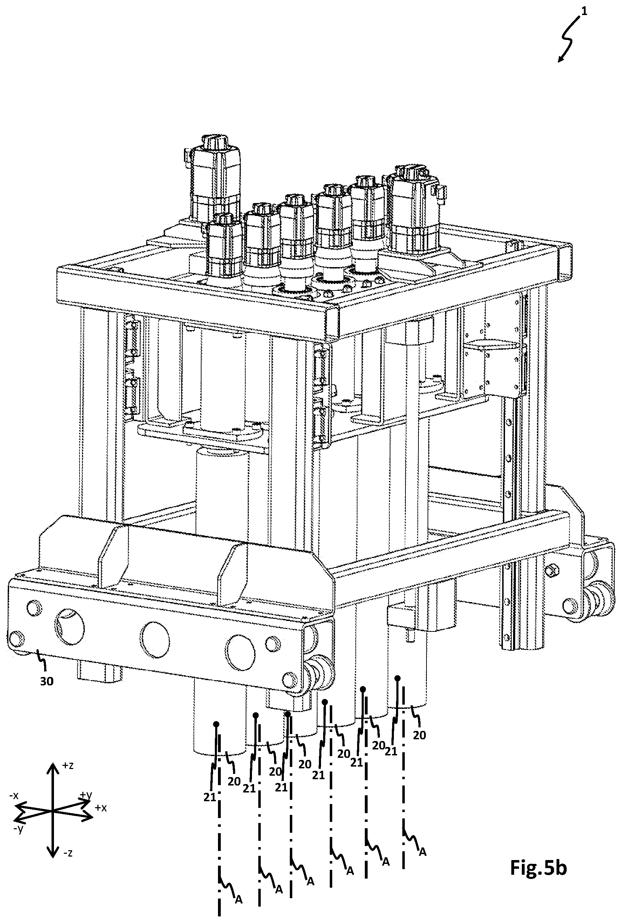

Fig.5a and Fig.5b show partial, perspective views of the apparatus (1) shown in Fig.4, from different aspects. Here, the channel (10) is omitted to emphasize an exemplary carrier (30) in communication with one or more cylinder(s) (20) (here: an exemplary plurality of cylinders (20)). Here, the one or more cylinder(s) (20) are in a state where they are translated away from the channel (10), in a direction orthogonal to the flow direction (FD). Thus, in such state, avoidance from contact between the cylinder(s) (20) and a respective waste stream is enabled, thereby facilitating the first translational movement of the cylinder(s) (20) along the flow direction (FD) relative to the channel (10).

Within this context, Fig.6 shows perspective view of the exemplary apparatus shown in Fig.1 to Fig.5b, showing a state where the one or more cylinder(s) (20) are at a second position with regard to the channel (10), said second position being different from the first position. Here, the cylinder(s) (20) are retracted away from the channel (30), for avoiding contact between the cylinder(s) (20) and a respective waste stream. Such positioning can be achieved for instance prior to returning of the cylinder(s) (20) to an upstream end of the channel (30) in accordance with the first translational movement, or at cleaning of the apparatus (1), or at an instance where the cylinder(s) (20) are just separated from the waste stream at a downstream end of the channel (30).

In a preferred embodiment, the one or more cylinder(s) (20) can be equipped with one or more scraping means (e.g., doctor blades, not shown) arranged to scrape the respective side surfaces (21), for separation of any excess pressed and ground waste stuck on respective side surfaces (21). This feature further enables crumbling and then reintroduction of such pressed waste into the stream; and also, aeration of such scraped waste. Upon such separation, any enzymes producing microorganisms present in the separated/scraped waste can be kept alive. The scraping means prevents accumulation of the pressed waste on side surfaces (21). Potential accumulation would augment pressure forces exerted towards the respective axes (A), which may result in mechanical damage on the apparatus (1). Hence, the scraping means also increase the service life of the apparatus (1) and minimizes the maintenance costs.

It is observed that, (in particular, for the case where the waste is a high solid load cellulosic waste) when a distance between the one or more cylinder(s)' side surface(s) (21) and corresponding complementary surface(s) (e.g., either on an adjacent cylinder (21) or on a corresponding plate (30)) orthogonal to a respective axis (A) is lower than 1 cm, difficulties start arising in introduction of waste in-between a side surface (21) and corresponding complementary surface. On the other hand, when said distance is higher than 4 cm, shear starts to be insufficient to achieve an effective grinding of the waste. Therefore, it is preferred that a distance between the one or more cylinder(s)' side surface(s) (21) and corresponding complementary surface(s) orthogonal to a respective axis (A) is within the range between 1 cm and 4 cm. It is observed that, in terms of increased effectiveness in hydrolysis of the high solid load cellulosic wastes, an optimum zone between facilitated introduction of waste in-between a side surface (21) and corresponding complementary surface and achieving an effective shear, is available when said distance is within the range between 1.5 cm and 3 cm.

It is further observed that in the case where the side surface(s) (21) and corresponding complementary surface(s) are provided with indentations that have sharp edges or sharp angles (e.g., higher than 5° over a full angle of 360°), cellulose adheres into the indentations and fill the same. This decreases the efficiency in grinding of cellulose. Therefore, it is preferred that any indentations on the side surface(s) (21) and/or corresponding complementary surface(s) have an angle that is up to 5°. For instance, the indentations can be rounded, curved, smoothened or wave-formed for avoiding sharp angles on the side surface(s) (21) and/or corresponding complementary surface(s).

Within the above-indicated contexts, the term "corresponding complementary surface(s)" corresponds to a respective grinding zone that is closest to a respective side surface (21) of a cylinder in terms of distance defined above.

### EXAMPLES:

The following examples are merely provided for proving the effectiveness of the proposed apparatus and method concept, without unduly limiting the scope of protection that is defined by the appended claims.

### EXAMPLE 1:

A cellulosic waste (here: paper sludge) is prepared such that the waste has a solid load within the range between 35 % (wt.) and 40 % (wt.) with regard to the total weight of the waste.

### EXAMPLE 2:

A first part of the waste is hydrolysed in the apparatus (1), with the cylinders (20) separated from the channel in order to simulate a prior art continuous hydrolysis process. Upon completion of a pre-determined residence time, a first hydrolysed waste is obtained. A first sample is taken from the first hydrolysed waste and diluted with water to obtain a first thinned sample that has a solid load of ca. 5 % (wt.) (here: 7.5 % (wt.)) with regard to the total weight of the first thinned sample. Viscosity of the first thinned sample is recorded as 20,000 centiPoises (which equals to 20,000 mPa.s). This viscosity is considered as an indicator of a usual extent of hydrolysis (or cellulose decomposition). Within the context of the present application, the measured viscosities are dynamic viscosities, and the viscosity measurements are made using Myr VP1000 series hand rotary viscometer in accordance with Brookfield method (ISO 2555).

### EXAMPLE 3:

A second part of the waste is hydrolysed in the apparatus (1) and in accordance with the method disclosed in the present application. The only difference of the hydrolysis process of EXAMPLE 3 from that of EXAMPLE 2 was the use of cylinders (20) and complementary surfaces for exertion of grinding. Upon completion of the pre-determined residence time, a second hydrolysed waste was obtained. A second sample is taken from the second hydrolysed waste and diluted with water to obtain a second thinned sample that has a solid load of ca. 5 % (wt.) (here: 7.5 % (wt.)) with regard to the total weight of the second thinned sample. Viscosity of the second thinned sample is recorded as 600 centiPoises. This viscosity is considered as an indicator of a surprisingly high extent of hydrolysis (or cellulose decomposition) when compared to the above-mentioned usual extent of hydrolysis.

### EXAMPLE 4:

Exemplary detailed information on the process mentioned in the EXAMPLE 3.
*1)* The cylinder(s) (20) were rotated at a very low rate (here: rotational speed): for instance, up to 10 rpm; The apparatus can be provided with one or more reduction gears (not shown) for determination/modulation of rotational speed of said one or more cylinders (20). Thus, a high extent of torque is achieved along with low rotational rate. As a result, the mechanical energy consumed by the apparatus can be substantially attributed to grinding.
*2)* The cylinder(s) (20) were translated along the flow direction (FD) at a very low speed: for instance, up to 50 cm/min.
*3)* Each pair of cylinders (20) in a row were arranged to promote the grounded waste in-between each other in the upstream direction opposite to the flow direction (FD).
*4)* It is contemplated that;
   *v)* when the cylinders (20) are protruded into the channel, the first translational movement in the downstream direction (+x) corresponds to promoting the waste in the flow direction (FW);
   *w)* a compound linear velocity resulting from movements of cooperating complementary surface(s) and side surface(s) (21) arranges a promotion of the waste in the flow direction (FW) or against the flow direction (FW); and
   *x)* a general linear velocity of the waste stream flowing through the channel (10) can be determined via manipulating the speed of first translational movement and the rotational speed of the cylinders (20); thus, the residence time can be manipulated.
*5)* When the cylinders (20) reach to a downstream end of the channel as a result of the first translational movement in the downstream direction (+x), the carrier (30) was operated to separate the cylinders (20) from the channel (10) by moving them in the upwards direction (+z); then the carrier (30) was further operated to translate the cylinders (20) in the upstream direction (-x); thus, the cylinders (20) were brought to e.g., an upstream end of the channel (10), and then re-introduced into the channel (10) by moving/translating them in the downwards direction (-z).
*6)* The cylinders (20) were translated again as in the item (2) above, corresponding to an iteration of the grinding of the waste and promoting of the same along the flow direction (FD); in the case where non-hydrolysed waste is fed from the upstream end and a corresponding amount of (at least partially-) hydrolysed waste is simultaneously streamed away (that is, taken or removed) from the downstream end, the apparatus (1) corresponds to a continuous hydrolysis reactor; (in a less preferred yet acceptable embodiment, the hydrolysed waste can be re-introduced into the channel (10) through the upstream end, and in such case, the apparatus (1) corresponds to a re-circulating or semi-batch hydrolysis reactor).
*7)* Within a pre-determined residence time, the iteration can be repeated (e.g., 100 to 200 times) to exert grinding onto the waste when streaming through the channel (10). With an increased number of rows, a rate of grinding can be increased even with a reduced number of repetition or iteration.
*8)* That the cellulosic waste has a solid load provides a high extent of internal friction coefficient to the waste, and reduces the fluidity of the same. So, effectiveness of grinding is increased with such high solid load. Within the context of the present application, for cellulosic wastes such as paper sludge are considered to have a high solid load when the solid load is within the range between 35 % (wt.) and 40 % (wt.) with regard to the total weight of said waste.
*9)* Viscosity as an indicator of extent of hydrolysis, refers to corresponding extent of decomposition of macromolecular cellulose structure at the end of the residence time; accordingly, as the hydrolysis in the channel (10) proceeds, the viscosity of the waste gradually decreases, resulting in a gradual decrease in effectiveness of grinding. Hence, the effectiveness in grinding (also the contribution for an effective hydrolysis) is at a maximum at the earlier stages of the operation of the apparatus (1), and gradually decreases throughout the residence time. The grinding is considered to be neither effective nor necessary at late stages of hydrolysis in which the cellulose is almost completely converted into glucose, and in which the viscosity reaches to a possible minimum value. Accordingly, the apparatus (1) and method according to the present invention can be considered to be most useful in a "partial hydrolysis" of high solid load cellulosic wastes, in particular, of wastes that contain crystalline cellulose. As an example to partial hydrolysis: a waste stream can have a mean glucose number of 10000 at entering the apparatus (1), and can be hydrolysed throughout the residence time until the mean glucose monomer number of cellulose molecules is decreased to a range between 5 and 5000. In other words, the hydrolysis is performed in a partial extent such that, at exiting the apparatus (1), the waste stream has a mean glucose monomer number of cellulose molecules at a range between 5 and 5000, preferably of up to 1000, for instance 100.
*10)* For effecting the hydrolysis during the residence time, the respective method can be considered to include the introduction of one or more enzymes (cellulases) or one or more cellulolytic microorganisms/bacteria (e.g., Cellulomonas fimi or Bacillus amyloliquefaciens) into the waste to be processed in the apparatus (1).
*11)* The apparatus (1) can include a heating means and temperature sensor(s), preferably in communication with a temperature controller, in order to adjust the waste temperature at an optimal level for hydrolysis.
*12)* Along with the grinding function, the cylinder(s) (20) and respective complementary surfaces further constitute a mixing means that contributes in enhancing the uniformity of the waste in the channel (10).

### Reference Signs:

- 1: apparatus
- 10: channel
- 20: cylinder
- 21: side surface
- 22: gap
- 23: pair (of cylinders)
- 30: carrier
- 40: plate
- A: axis
- FD: flow direction
- +x: downstream direction
- -x: upstream direction
- -y/+y: lateral orientation
- +z: upwards direction
- -z: downwards direction

## Claims

1. An apparatus (1) for use in cellulose hydrolysis, comprising:
- a channel (10) for guiding a stream of cellulose-containing waste in a flow direction (FD) and for supporting the stream by a bottom of the channel (10) against gravity (g) which is orthogonal to the flow direction (FD) when the apparatus (1) is in use;
- one or more cylinder(s) (20) arranged to rotate around an axis (A), such that, when rotated, one or more side surfaces (21) of said cylinder(s) (20) exhibit a linear velocity vector orthogonal to said axis (A);
- one or more complementary surface(s) that face said side surfaces (21), such that, when the cylinder(s) (20) are rotated, the side surfaces (21) and complementary surfaces cooperate in grinding the waste that flows in the flow direction (FD).

2. The apparatus according to claim 1, wherein the one or more cylinder(s) (20) are arranged to have their respective axes (A) extending along the gravity vector (g) and orthogonal to the flow direction (FD) when the apparatus (1) is in use.

3. The apparatus according to any of claims 1 or 2, wherein said one or more cylinder(s) (20) are each arranged to rotate in a respective rotational direction(s) to prompt the ground waste opposite to the flow direction (FD).

4. The apparatus according to any of claims 1 to 3, wherein said one or more cylinder(s) (20) are each arranged to rotate such that respective side surfaces (21) thereof exhibit a relative linear velocity higher than zero with respect to said complementary surfaces.

5. The apparatus according to any of claims 1 to 4, wherein the side surfaces (21) of said one or more cylinders (20) are provided with indentations.

6. The apparatus according to any of claims 1 to 5, comprising one or more plate(s) (40) arranged to face said one or more cylinder(s) (20) for serving as complementary surface(s) to the side surfaces (21) of respective cylinder(s) (20).

7. The apparatus according to any of claims 1 to 5, comprising a plurality of cylinders (20) adjacent to each other, such that opposing side surfaces (21) of a pair of said cylinders (20) adjacent to each other serve as complementary surfaces to each other.

8. The apparatus according to claim 7, wherein one or more pairs of said plurality of cylinders (20) are arranged to rotate in opposite rotational directions, such that their side surfaces (21) opposing each other both have linear velocities opposite to the flow direction (FD).

9. The apparatus according to claim 7 or 8, wherein the plurality of cylinders (20) adjacent to each other are arranged to exhibit different linear velocities with respect to the opposing side surfaces (21) of each other.

10. The apparatus according to any of claims 1 to 9, comprising a carrier (30) arranged to provide a first translational movement to the one or more cylinder(s) (20) relative to the channel (10) along the flow direction (FD).

11. The apparatus according to claim 10, wherein the carrier (30) is arranged to provide that the first translational movement of the one or more cylinder(s) (20) is orthogonal to their respective axes (A).

12. A method for cellulose hydrolysis, including the following actions:
- guiding a stream of cellulose-containing waste in a flow direction (FD) along a channel (10), supporting the stream by a bottom of the channel (10) against gravity (g) which is orthogonal to the flow direction (FD); and
- grinding the waste that flows in the flow direction (FD), between one or more side surface(s) (21) of one or more cylinder(s) (20) that rotate around an axis (A) and one or more complementary surfaces that cooperate with respective side surface(s) (21), by arranging said side surface(s) (21) to exhibit a linear velocity vector orthogonal to the respective axis (A).

13. The method according to claim 12, wherein the method includes coupling of opposing side surfaces (21) of a plurality of such cylinders (20) adjacent to each other, as complementary surfaces with regard to each other.

14. The method according to any of claims 12 or 13, further including provision of a first translational movement to the one or more cylinder(s) (20) relative to the channel (10) along the flow direction (FD).

15. The method according to any of claims 12 to 14, further including provision of a second translational movement to the one or more cylinder(s) (20) relative to the channel (10) orthogonal to the flow direction (FD).

## Patentansprüche

1. Vorrichtung (1) zur Verwendung bei der Zellulosehydrolyse, umfassend:
- einen Kanal (10) zum Führen eines Stroms von zellulosehaltigem Abfall in einer Strömungsrichtung (FD) und zum Abstützen des Stroms durch einen Boden des Kanals (10) gegen die Schwerkraft (g), die senkrecht zu der Strömungsrichtung (FD) verläuft, wenn die Vorrichtung (1) in Gebrauch ist;
- einen oder mehrere Zylinder (20), die dazu angeordnet sind, sich um eine Achse (A) zu drehen, sodass bei einer Drehung eine oder mehrere Seitenflächen (21) des oder der Zylinder (20) einen linearen Geschwindigkeitsvektor aufweisen, der senkrecht zu der Achse (A) verläuft;
- eine oder mehrere Gegenflächen, die den Seitenflächen (21) gegenüberliegen, sodass bei einer Drehung des oder der Zylinder (20) die Seitenflächen (21) und die Gegenflächen zusammenwirken, um den in der Strömungsrichtung (FD) strömenden Abfall zu zermahlen.

2. Vorrichtung nach Anspruch 1, wobei der oder die mehrere Zylinder (20) so angeordnet sind, dass sich ihre jeweiligen Achsen (A) entlang des Schwerkraftvektors (g) und senkrecht zu der Strömungsrichtung (FD) erstrecken, wenn die Vorrichtung (1) in Gebrauch ist.

3. Vorrichtung nach einem der Ansprüche 1 oder 2, wobei der oder die mehrere Zylinder (20) jeweils dazu angeordnet sind, sich in einer jeweiligen Drehrichtung oder in jeweiligen Drehrichtungen zu drehen, um den zermahlenen Abfall entgegen der Strömungsrichtung (FD) zu fördern.

4. Vorrichtung nach einem der Ansprüche 1 bis 3, wobei der oder die mehrere Zylinder (20) jeweils dazu angeordnet sind, sich so zu drehen, dass ihre jeweiligen Seitenflächen (21) gegenüber den Gegenflächen eine relative lineare Geschwindigkeit größer als null aufweisen.

5. Vorrichtung nach einem der Ansprüche 1 bis 4, wobei die Seitenflächen (21) des oder der mehreren Zylinder (20) mit Vertiefungen versehen sind.

6. Vorrichtung nach einem der Ansprüche 1 bis 5, umfassend eine oder mehrere Platten (40), die dazu angeordnet sind, dem einen oder den mehreren Zylindern (20) gegenüberzuliegen, um als Gegenfläche(n) zu den Seitenflächen (21) der jeweiligen Zylinder (20) zu dienen.

7. Vorrichtung nach einem der Ansprüche 1 bis 5, umfassend eine Mehrzahl von aneinander angrenzenden Zylindern (20), sodass einander gegenüberliegende Seitenflächen (21) eines Paars von aneinander angrenzenden Zylindern (20) als Gegenflächen zueinander dienen.

8. Vorrichtung nach Anspruch 7, wobei ein oder mehrere Paare der Mehrzahl von Zylindern (20) dazu angeordnet sind, sich in entgegengesetzten Drehrichtungen zu drehen, sodass ihre einander gegenüberliegenden Seitenflächen (21) beide lineare Geschwindigkeiten entgegen der Strömungsrichtung (FD) aufweisen.

9. Vorrichtung nach Anspruch 7 oder 8, wobei die Mehrzahl von aneinander angrenzenden Zylindern (20) dazu angeordnet ist, mit Bezug auf die einander gegenüberliegenden Seitenflächen (21) voneinander verschiedene lineare Geschwindigkeiten aufzuweisen.

10. Vorrichtung nach einem der Ansprüche 1 bis 9, umfassend einen Träger (30), der dazu angeordnet ist, dem einen oder den mehreren Zylindern (20) relativ zu dem Kanal (10) entlang der Strömungsrichtung (FD) eine erste translatorische Bewegung zu verleihen.

11. Vorrichtung nach Anspruch 10, wobei der Träger (30) dazu angeordnet ist, zu bewirken, dass die erste translatorische Bewegung des oder der mehreren Zylinder (20) senkrecht zu deren jeweiligen Achsen (A) verläuft.

12. Verfahren zur Zellulosehydrolyse, umfassend die folgenden Schritte:
- Führen eines Stroms von zellulosehaltigem Abfall in einer Strömungsrichtung (FD) entlang eines Kanals (10), wobei der Strom durch einen Boden des Kanals (10) gegen die Schwerkraft (g) abgestützt wird, die senkrecht zu der Strömungsrichtung (FD) verläuft; und
- Zermahlen des in der Strömungsrichtung (FD) strömenden Abfalls zwischen einer oder mehreren Seitenflächen (21) eines oder mehrerer Zylinder (20), die sich um eine Achse (A) drehen, und einer oder mehreren Gegenflächen, die mit den jeweiligen Seitenflächen (21) zusammenwirken, indem bewirkt wird, dass die Seitenfläche(n) (21) einen linearen Geschwindigkeitsvektor aufweisen, der senkrecht zu der jeweiligen Achse (A) verläuft.

13. Verfahren nach Anspruch 12, wobei das Verfahren das Koppeln einander gegenüberliegender Seitenflächen (21) einer Mehrzahl solcher aneinander angrenzender Zylinder (20) als Gegenflächen zueinander umfasst.

14. Verfahren nach einem der Ansprüche 12 oder 13, ferner umfassend das Verleihen einer ersten translatorischen Bewegung an den oder die mehreren Zylinder (20) relativ zu dem Kanal (10) entlang der Strömungsrichtung (FD).

15. Verfahren nach einem der Ansprüche 12 bis 14, ferner umfassend das Verleihen einer zweiten translatorischen Bewegung an den oder die mehreren Zylinder (20) relativ zu dem Kanal (10) senkrecht zu der Strömungsrichtung (FD).

## Revendications

1. Appareil (1) destiné à être utilisé dans l'hydrolyse de la cellulose, comprenant :
- un canal (10) destiné à guider un flux de déchets contenant de la cellulose dans une direction d'écoulement (FD) et à soutenir le flux au moyen d'un fond du canal (10) pour lutter contre la gravité (g) qui est orthogonale à la direction d'écoulement (FD) lorsque l'appareil (1) est utilisé ;
- un ou plusieurs cylindres (20) agencés pour tourner autour d'un axe (A), de sorte que, lorsqu'ils sont mis en rotation, une ou plusieurs surfaces latérales (21) du ou desdits cylindres (20) présentent un vecteur de vitesse linéaire orthogonal audit axe (A) ;
- une ou plusieurs surfaces complémentaires qui font face auxdites surfaces latérales (21), de sorte que, lorsque le ou les cylindres (20) sont mis en rotation, les surfaces latérales (21) et les surfaces complémentaires coopèrent pour broyer les déchets qui s'écoulent dans la direction d'écoulement (FD).

2. Appareil selon la revendication 1, dans lequel les un ou plusieurs cylindres (20) sont agencés de façon à ce que leurs axes (A) respectifs s'étendent le long du vecteur de gravité (g) et soient orthogonaux à la direction d'écoulement (FD) lorsque l'appareil (1) est utilisé.

3. Appareil selon l'une quelconque des revendications 1 ou 2, dans lequel lesdits un ou plusieurs cylindres (20) sont chacun agencés pour tourner dans une ou plusieurs directions de rotation respectives pour amener les déchets broyés à l'opposé de la direction d'écoulement (FD).

4. Appareil selon l'une quelconque des revendications 1 à 3, dans lequel lesdits un ou plusieurs cylindres (20) sont chacun agencés pour tourner de sorte que leurs surfaces latérales (21) respectives présentent une vitesse linéaire relative supérieure à zéro par rapport auxdites surfaces complémentaires.

5. Appareil selon l'une quelconque des revendications 1 à 4, dans lequel les surfaces latérales (21) desdits un ou plusieurs cylindres (20) sont dotées d'empreintes.

6. Appareil selon l'une quelconque des revendications 1 à 5, comprenant une ou plusieurs plaques (40) agencées pour faire face auxdits un ou plusieurs cylindres (20) pour servir de surface(s) complémentaire(s) aux surfaces latérales (21) du ou des cylindres (20) respectifs.

7. Appareil selon l'une quelconque des revendications 1 à 5, comprenant une pluralité de cylindres (20) adjacents les uns aux autres, de sorte que des surfaces latérales opposées (21) d'une paire desdits cylindres (20) adjacents les uns aux autres servent de surfaces complémentaires les unes aux autres.

8. Appareil selon la revendication 7, dans lequel une ou plusieurs paires de ladite pluralité de cylindres (20) sont agencées pour tourner dans des directions de rotation opposées, de sorte que leurs surfaces latérales (21) opposées l'une à l'autre présentent toutes deux des vitesses linéaires opposées à la direction d'écoulement (FD).

9. Appareil selon la revendication 7 ou 8, dans lequel la pluralité de cylindres (20) adjacents les uns aux autres sont agencés pour présenter des vitesses linéaires différentes par rapport aux surfaces latérales (21) opposées les unes aux autres.

10. Appareil selon l'une quelconque des revendications 1 à 9, comprenant un support (30) agencé pour fournir un premier mouvement de translation aux un ou plusieurs cylindres (20) par rapport au canal (10) le long de la direction d'écoulement (FD).

11. Appareil selon la revendication 10, dans lequel le support (30) est agencé pour permettre au premier mouvement de translation des un ou plusieurs cylindres (20) d'être orthogonal à leurs axes (A) respectifs.

12. Procédé d'hydrolyse de la cellulose comportant les actions suivantes :
- le guidage d'un flux de déchets contenant de la cellulose dans une direction d'écoulement (FD) le long d'un canal (10), le soutien du flux au moyen d'un fond du canal (10) pour lutter contre la gravité (g) qui est orthogonale à la direction d'écoulement (FD) ; et
- le broyage des déchets qui s'écoulent dans la direction d'écoulement (FD), entre une ou plusieurs surfaces latérales (21) d'un ou plusieurs cylindres (20) qui tournent autour d'un axe (A) et une ou plusieurs surfaces complémentaires qui coopèrent avec la ou les surfaces latérales (21) respectives, en agençant ladite ou lesdites surfaces latérales (21) pour qu'elles présentent un vecteur de vitesse linéaire orthogonal à l'axe (A) respectif.

13. Procédé selon la revendication 12, dans lequel le procédé comporte l'accouplement de surfaces latérales (21) opposées d'une pluralité de tels cylindres (20) adjacents les uns aux autres, en tant que surfaces complémentaires les unes par rapport aux autres.

14. Procédé selon l'une quelconque des revendications 12 ou 13, comportant en outre la fourniture d'un premier mouvement de translation aux un ou plusieurs cylindres (20) par rapport au canal (10) le long de la direction d'écoulement (FD).

15. Procédé selon l'une quelconque des revendications 12 à 14, comportant en outre la fourniture d'un deuxième mouvement de translation aux un ou plusieurs cylindres (20) par rapport au canal (10) orthogonal à la direction d'écoulement (FD).
